# EUROPEAN PATENT APPLICATION

(11) **EP 2 708 525 A1**
(43) Date of publication of application: **19.03.2014**
(21) Application number: 12183984.9
(22) Date of filing: 12.09.2012
(51) Int. Cl.: C07C 29/62, C07C 29/76, C07C 31/36, C07D 301/26, C07D 303/08

(54) **Process of stripping a liquid medium**

(71) Applicant: Solvay SA, 1120 Bruxelles (BE)
(72) Inventor: Gilbeau, Patrick, 7090 Braine-le-Comte (BE); Fouchet, Bruno, Qing Pu Shanghai 201702 (CN)
(74) Representative: Vande Gucht, Anne

(57) **Abstract**

Process of stripping a liquid medium containing at least one oligomer of glycerol, wherein a stripping agent comprising hydrogen chloride is used at a temperature higher than 120 °C.

## Description

The present invention relates to a process of stripping a liquid medium.

The present invention relates more specifically to a process of stripping a liquid medium comprising an effluent generated by a process for converting glycerol and/or esters thereof into chlorohydrins.

Dichlorohydrins of glycerol are useful in preparing epoxides such as epichlorohydrin. Epichlorohydrin is a widely used precursor to epoxy resins. Epichlorohydrin is a monomer which is commonly used for the alkylation of para-bisphenol A. The resultant diepoxide, either as a free monomer or oligomeric diepoxide, may be processed to high molecular weight resins which are used for example in electrical laminates, can coatings, automotive topcoats and clearcoats.

Glycerin is considered to be a low-cost, renewable feedstock that is a coproduct of the biodiesel process for making fuel. It is known that other renewable feedstocks such as fructose, glucose and sorbitol can be hydrogenolized to produce mixtures of vicinal diols and triols, such as glycerin, ethylene glycol, 1,2-propylene glycol, 1,3-propylene glycol and the like. With abundant and low cost glycerin or mixed glycols, economically attractive processes for recovering dichlorohydrins from effluents produced by the above processes are desired.

International application WO 2008/125004 discloses a process for recovering dichlorohydrin(s) from a residue resulting of the distillation or fractionation of a mixture obtained by hydrochlorination of glycerol and/or of esters thereof, by stripping with a stripping agent, preferably selected from steam, nitrogen, methane, carbon dioxide and mixtures thereof.

While such process is capable of recovering some of the dichlorohydrins present in the mixture, opportunities for improvement remain, in particular, for the recovery of chlorohydrins that might be included as glycerol oligomers in distillation residues.

The invention therefore relates in a first embodiment to a process of stripping a liquid medium containing at least one oligomer of glycerol wherein a stripping agent comprising hydrogen chloride is used at a temperature higher than 120 °C.

In the process according to the invention, the oligomer of glycerol can be at least partially chlorinated, esterificated, or chlorinated and esterificated. It is preferably partially chlorinated and/or esterificated.

One of the essential features of the present invention lies in the combined uses of a stripping agent comprising hydrogen chloride and of a temperature of stripping of at least 120 °C, with the following advantages:
● Use of a stripping agent which is one of the raw materials used in the process for manufacturing dichloropropanol from glycerol, recyclable in said manufacturing step, therefore lowering the complexity of the overall process,
● Increase of the recovery of valuable materials from the liquid medium.

Without willing to be tied by any theory, it is indeed believed that the use of a stripping agent comprising hydrogen chloride combined with a high stripping temperature can lead to de-oligomerization and de-esterification reactions, and to the subsequent conversion of polymeric compounds into valuable easily strippable molecules, like glycerol, monochloropropanediol and dichloropropanol.

By monochloropropanediol, one intends to denote 3-chloro-1,2-propanediol, 2-chloro-1,3-propanediol or a mixture of them. By ichloropropanol, one intends to denote 1,3-dichloro-2-propanol, 2,3-dichloro-1-propanol or a mixture of them. Dichlorohydrin or dichlorohydrins of glycerol can be used to designate dichloropropanol.

In the process according to the invention, the liquid reaction medium contains at least one glycerol oligomer. The oligomer of glycerol is preferably selected from the group consisting of oligomers of glycerol of linear structure, of branched structure, of cyclic structure and of mixtures of at least two of these oligomers.

The expression "oligomers of linear structure" is understood to mean oligomers in which all the carbon atoms are located in one and the same chain of atoms, which is not a ring.

The expression "oligomers of branched structure" is understood to mean oligomers for which the carbon atoms are located in at least two chains of atoms.

The expression "oligomer of cyclic structure " is understood to mean a cyclic compound resulting from condensation reactions between at least two glycerol molecules, i.e. a compound resulting from condensation reactions between at least two glycerol molecules and the chemical structure of which contains at least one cycle or ring.

In the process according to the invention, the glycerol oligomer is generally a compound resulting from condensation reactions between at least 2 glycerol molecules (dimer), often at least 3 glycerol molecules (trimer), and frequently at least 4 glycerol molecules (tetramer). In the process according to the invention, the glycerol oligomer is generally a compound resulting from condensation reactions between at most 10 glycerol molecules (decamer), often at most 9 glycerol molecules (nonamer), frequently at most 8 glycerol molecules (octamer) and more specifically at most 7 glycerol molecules (heptamer).

In the process according to the invention, the glycerol oligomer of linear structure and the glycerol oligomer of branched structure are independently and frequently a glycerol dimer.

In the process according to the invention, the glycerol dimer of linear structure and the glycerol dimer of branched structure are often a mixture of the dimer of linear structure, and of at least one dimer of branched structure.

The non cyclic oligomer of glycerol is often a mixture of at least two of the compounds selected from the group consisting of 3-(2,3-dihydroxypropoxy)-propane-1,2-diol (linear oligomer of glycerol), 3-(2-hydroxy-1-hydroxymethylethoxy)propane-1,2-diol (mono branched oligomer of glycerol) and 2-(2-hydroxy-1-hydroxymethylethoxy)propane-1,3-diol) (di branched oligomer of glycerol).

In the process according to the invention, the cyclic oligomer of glycerol is usually an oligomer of glycerol for which at least some of the carbon atoms are located in the at least one ring of the chemical structure. The number of atoms constituting the ring is generally greater than or equal to 6, often greater than or equal to 7 and sometimes greater than or equal to 8. The number of atoms constituting the ring is generally less than or equal to 20. The ring generally comprises at least two oxygen atoms and often 2 oxygen atoms. Cyclic oligomers of glycerol containing a single ring constituted of 6 atoms, of which 2 of the atoms are oxygen atoms, are particularly suitable. Cyclic oligomers of glycerol containing a single ring constituted of 7 atoms, of which 2 of the atoms are oxygen atoms, are particularly convenient. Cyclic oligomers of glycerol containing a single ring constituted of 8 atoms, of which 2 of the atoms are oxygen atoms, are also particularly suitable.

In the process according to the invention, the cyclic oligomer of glycerol is preferably chosen from the group consisting of cyclic dimers of glycerol, cyclic trimers of glycerol, cyclic tetramers of glycerol, and mixtures of at least two of these glycerol oligomers.

In the process according to the invention, the cyclic oligomer of glycerol is often a cyclic compound resulting from condensation reactions between two glycerol molecules, i.e., a cyclic dimer of glycerol.

The dimer of cyclic structure generally comprises at least one ring and often only one ring. The ring generally comprises 6 atoms, often 7 atoms and frequently 8 atoms, of which two atoms are oxygen atoms and the remainder are carbon atoms.

In the process according to the invention, the cyclic dimer of glycerol usually comprises at least one of the compounds selected from the group consisting of cis- and trans-2,5-bis-(hydroxymethyl)-1,4-dioxane, cis- and trans-2,6-bis(hydroxymethyl)-1,4-dioxane, cis- and trans-6-hydroxy-2-hydroxymethyl-1,4-dioxepane, and cis- and trans-3,7-dihydroxy-1,5-dioxocane, and any mixture of at least two of them.

In the process according to the invention, the cyclic dimer of glycerol is often a mixture comprising all the preceding isomers.

In the process according to the invention, the cyclic dimer of glycerol is often a mixture consisting essentially of all the preceding isomers.

In the rest of the document, glycerol oligomers will also be called polyglycerols, and glycerol dimers, trimers,tetramers, etc. will also be called diglycerols, triglycerols, tetraglycerols, etc..

In the process according to the invention, the oligomer of glycerol can be chlorinated and/or esterificated.

The expression at least partially chlorinated oligomer of glycerol is understood to mean an oligomer of glycerol as defined above of which at least one hydroxyl group (-OH) has been replaced by a chlorine atom. In such chlorinated oligomer of glycerol, at most all the hydroxyl groups have been replaced by a chlorine atom.

A chlorinated oligomer of glycerol can result from the reaction between hydrogen chloride with an oligomer of glycerol, from the condensation of glycerol with at least one of monochloropropanediol, dichloropropanol and a chlorinated oligomer of glycerol, from the condensation of an oligomer of glycerol with at least one of monochloropropanediol, dichloropropanol and a chlorinated oligomer of glycerol, from the condensation of at least two compounds, identical of different, selected from the group consisting of monochloropropanediol, dichloropropanol and a chlorinated oligomer of glycerol, and from any combination thereof.

The chlorinated oligomers of glycerol are more specifically chosen from the group consisting of 3-(3-chloro-2-hydroxypropoxy)-propane-1,2-diol, 3-(2-chloro-3-hydroxypropoxy)-propane-1,2-diol, 2-(3-chloro-2-hydroxypropoxy)-1,3-propanediol, 3-(2-chloro-1-hydroxymethylethoxy)propane-1,2-diol, 2-(2-chloro-1-hydroxymethylethoxy)propane-1,3-diol), 1-chloro-2-hydroxy-3-(3-chloro-2-hydroxypropoxy)-propane, 2-chloro-1-hydroxy-3-(3-chloro-2-hydroxypropoxy)-propane, 1-chloro-2-hydroxy-3-(2-chloro-1-hydroxymethylethoxy)propane, 2-chloro-1-hydroxy-3-(2-chloro-1-hydroxymethylethoxy)propane, 1-chloro-3-hydroxy-2-(2-chloro-1-hydroxymethylethoxy)propane, cis- and trans-2-chloromethyl-5-hydroxymethyl-1,4-dioxane, cis- and trans-2-chloromethyl-6-hydroxymethyl-1,4-dioxane, cis- and trans-6-chloro-2-hydroxymethyl-1,4-dioxepane, cis- and trans-6-hydroxy-2-chloromethyl-1,4-dioxepane, cis- and trans-3-chloro-7-hydroxy-1,5-dioxocane, cis- and trans-2,5-bis-(chloromethyl)-1,4-dioxane, cis- and trans-2,6-bis(chloromethyl)-1,4-dioxane, cis- and trans-6-chloro-2-chloromethyl-1,4-dioxepane, and cis- and trans-3,7-dichloro-1,5-dioxocane.

The expression at least partially esterificated oligomer of glycerol is understood to mean an oligomer of glycerol as defined above of which at least one hydroxyl group (-OH) has been replaced by the conjugated basic form of an acid, with the exception of hydrogen chloride. In such chlorinated oligomer of glycerol, at most all the hydroxyl groups have been replaced by the conjugated basic form of an acid, with the exception of hydrogen chloride. The acid can be a inorganic acid, an organic acid, or a mixture thereof. The inorganic acid is preferably selected from the group consisting of sulfuric acid, phosphoric acid, phosphorous acid, heteropolyacids, and mixture thereof. The acid is preferably an organic acid selected from the group consisting of a carboxylic acid, a sulfonic acid, a sulfinic acid, a phosphonic acid, a phosphinic acid, and mixture thereof. The acid is more preferably carboxylic acid. The esterificated oligomer of glycerol is preferably an ester of a carboxylic acid and of the glycerol oligomer. In this case, at least one hydroxyl group (-OH) of the glycerol oligomer has been replaced has been replaced by a carboxy group (RC=OO-) and at most all the hydroxyl groups have been replaced by such a group.

An esterificated oligomer of glycerol with a carboxylic acid can result from the reaction between a carboxylic acid RCOOH with an oligomer of glycerol, from the condensation of glycerol with at least one of a monoester of glycerol, a diester of glycerol and an esterificated oligomer of glycerol, from the condensation of esterificated oligomers, and from any combination thereof.

The R- group can be an alkyl, an alicyclic aliphatic alkyl, an arylalkyl or an alkylaryl group, linear or branched. The R group contains a number of carbon atoms which is generally higher than or equal to 1, often higher than or equal to 2 and frequently higher than or equal to 3. That number of carbon atoms is generally lower than or equal to 20, often lower than or equal to 15 and frequently lower than or equal to 10. The R- group may also contain a least one heteroatom such as chlorine, oxygen, sulfur and nitrogen for instance.

The carboxylic acid RCOOH can be a monocarboxylic acid or a polycarboxylic acid, such as disclosed in application WO 2005/054167 in the name of Solvay S.A., the content of which is incorporated by reference, more specifically at page 7, lines 11-35. The monocarboxylic acid may be a light carboxylic acid such as formic acid, acetic acid, propionic acid or butyric acid, or a fatty acid. The mono carboxylic acid is preferably acetic acid. The carboxylic acid is preferably a polycarboxylic acid and more preferably a dicarboxylic acid. The dicarboxylic acid is preferably selected from the group consisting of succinic acid, glutaric acid, adipic acid and mixtures thereof. Glutaric acid and adipic acid are preferred and adipic acid is more preferred.

The expression partially chlorinated and esterificated oligomer of glycerol is understood to mean an oligomer of glycerol as defined above of which at least one hydroxyl group (-OH) has been replaced by a chlorine atom and of which at least one hydroxyl group (-OH) has been replaced by the conjugated basic form of an acid, with the exception of hydrogen chloride, preferably by a carboxy group (RC=OO-), as defined above. The chlorinated and esterificated oligomer of glycerol can be obtained by any combination of the reactions disclosed above.

In the process according to the invention, the content of the oligomer of glycerol in the liquid medium before stripping expressed as g of glycerol per kg of liquid medium is usually greater than or equal to 0.005 g/kg, usually greater than or equal to 0.05 g/kg, commonly greater than or equal to 0.1 g/kg, often greater than or equal to 1 g/kg, frequently greater than or equal to 5 g/kg, and particularly greater than or equal to 10 g/kg. This oligomer content is generally less than or equal to 250 g/kg, usually less than or equal to 200 g/kg, commonly less than or equal to 150 g/kg, often less than or equal to 125 g/kg, frequently less than or equal to 100 g/kg and particularly less than or equal to 80 g/kg.

The same applies to the content of the oligomer of glycerol chlorinated and/or esterificated. The same applies also specifically to the sum of the following oligomers of glycerol, dimers of glycerol, dimers of glycerol dichlorinated, monocarboxylate of dichlorinated dimers of glycerol and monocarboxylates of monochlorinated dimers of glycerol, and more specifically to the sum of the said species where the carboxylate is one of acetate, succinate, glutarate, or adipate, and most specifically to the sum of the said species where the carboxylate is adipate.

The same applies to the content of any of the specific oligomers of glycerol, possibly chlorinated and/or esterificated described above.

In the process according to the invention, the liquid mixture may comprise in addition at least one of glycerol, a glycerol chlorohydrin, and esters thereof. The glycerol chlorohydrin can be monochloropropanediol or dichloropropanol.

In the process according to the invention, the esters of glycerol, of the glycerol oligomers, of the chlorinated oligomers of glycerol, of monochloropropanediol and of dichloropropanol are preferably carboxylic acid esters. The carboxylic acids are as described above.

In the process according to the invention, the content of glycerol in the liquid medium before stripping is generally greater than or equal to 0.001 g/kg, usually greater than or equal to 0.01 g/kg, commonly greater than or equal to 0.1 g/kg, often greater than or equal to 1 g/kg, frequently greater than or equal to 2 g/kg, and particularly greater than or equal to 5 g/kg. This content of glycerol is generally less than or equal to 100 g/kg, usually less than or equal to 80 g/kg, commonly less than or equal to 60 g/kg, often less than or equal to 50 g/kg, frequently less than or equal to 20 g/kg and particularly less than or equal to 10 g/kg..

In the process according to the invention, the content of glycerol ester, in particular of monocarboxlate of glycerol in the liquid medium before stripping is generally greater than or equal to 0.01 g of glycerol per kg of liquid medium, usually greater than or equal to 0.1 g/kg, habitually greater than or equal to 0.5 g/kg, in a lot of cases greater than or equal to 1 g/kg and often greater than or equal to 2 g/kg. This content is generally less than or equal to 70 g/kg, often less than or equal to 80 g/kg, frequently less than or equal to 90 g/kg and in a lot of cases less than or equal to 100g/kg.

In the process according to the invention, the content of glycerol chlorohydrin in the liquid medium before stripping is generally greater than or equal to 100 g of chlorohydrin per kg of liquid medium, usually greater than or equal to 200 g/kg, habitually greater than or equal to 300 g/kg, in a lot of cases greater than or equal to 400 g/kg and often greater than or equal to 500 g/kg. This content is generally less than or equal to 950 g/kg, often less than or equal to 900 g/kg, frequently less than or equal to 800 g/kg and in a lot of cases less than or equal to 700 g/kg.

In the process according to the invention, the content of glycerol chlorohydrin ester, in particular monocarboxylate of chlorohydrin ester, in the liquid medium before stripping is generally greater than or equal to 100 g of chlorohydrin per kg of liquid medium, usually greater than or equal to 200 g/kg, habitually greater than or equal to 300 g/kg, in a lot of cases greater than or equal to 400 g/kg and often greater than or equal to 500 g/kg. This content is generally less than or equal to 950 g/kg, often less than or equal to 900 g/kg, frequently less than or equal to 800 g/kg and in a lot of cases less than or equal to 700 g/kg.

In the process according to the invention, the liquid medium may additionally contain at least one compound chosen from water, alcohols, aldehydes, ketones, carboxylic acids, carboxylic acid esters, ethers, halogenated hydrocarbons, epoxides, metals, in the metallic state or in the salt state, and mixtures of at least two of them. The chlorohydrins are not considered to be alcohols or halogenated hydrocarbons or ethers or esters. This case is encountered often when the liquid medium originates from the purge of a process for the manufacture of dichloropropanol by hydrochlorination of a glycerol.

The content of water in the liquid medium is generally greater than or equal to 0.01 g/kg, usually greater than or equal to 0.1 g/kg, commonly greater than or equal to 1 g/kg, often greater than or equal to 5 g/kg, frequently greater than or equal to 10 g/kg, and particularly greater than or equal to 20 g/kg. This water content is generally less than or equal to 300 g/kg, usually less than or equal to 200 g/kg, commonly less than or equal to 150 g/kg, often less than or equal to 100 g/kg, frequently less than or equal to 70 g/kg and particularly less than or equal to 50 g/kg.

The carboxylic acids may be as defined above. The carboxylic acid is preferably chosen from monocarboxylic acids, polycarboxylic acids, often dicarboxylic acids, and mixtures of at least two of them. Acetic acid is an example of a monocarboxylic acid. Adipic acid is an example of a dicarboxylic acid.

The content of carboxylic acid in the liquid medium is generally greater than or equal to 0.001 g/kg, usually greater than or equal to 0.01 g/kg, commonly greater than or equal to 0.1 g/kg, often greater than or equal to 0.5 g/kg, frequently greater than or equal to 1 g/kg, and particularly greater than or equal to 5 g/kg. This carboxylic acid content is generally less than or equal to 100 g/kg, usually less than or equal to 80 g/kg, commonly less than or equal to 60 g/kg, often less than or equal to 40 g/kg, frequently less than or equal to 20 g/kg and particularly less than or equal to 10 g/kg.

The carboxylic acid esters may be as defined above. They are preferably chosen from the esters of the aforementioned acids with chlorohydrins and alcohols. In particular, these esters may be chosen from the adipates of glycerol, of diglycerol, of monochloropropanediol, of dichloropropanol, and mixtures of at least two of them.

The content of carboxylic acid ester in the liquid medium is generally greater than or equal to 0.01 g/kg, usually greater than or equal to 0.1 g/kg, commonly greater than or equal to 1 g/kg, often greater than or equal to 5 g/kg, frequently greater than or equal to 10 g/kg, and particularly greater than or equal to 15 g/kg. This carboxylic acid ester content is generally less than or equal to 500 g/kg, usually less than or equal to 300 g/kg, commonly less than or equal to 150 g/kg, often less than or equal to 100 g/kg, frequently less than or equal to 50 g/kg and particularly less than or equal to 20 g/kg.

In the process according to the invention, the stripping agent may comprise in addition at least one compound selected from the group consisting of steam, methane, carbon dioxide, nitrogen, air, oxygen depleted air, and any mixture thereof.

In the process according to the invention, the content of hydrogen chloride in the stripping agent is usually higher than or equal to 0.1 percent per volume, preferably higher than or equal to 1 percent per volume, more preferably higher than or equal to 5 percent per volume and most preferably preferably higher than or equal to 10 percent per volume. This content is usually lower than or equal to 99.9* percent per volume, preferably lower than or equal to 99 percent per volume, more preferably lower than or equal to 95 percent per volume and most preferably preferably lower than or equal to 90 percent per volume.

A stripping agent consisting essentially of hydrogen chloride is preferred.

In the process according to the invention, the stripping agent may contain steam in an amount usually higher than or equal to 0.1 percent per volume, preferably higher than or equal to 1 percent per volume, more preferably higher than or equal to 5 percent per volume and most preferably preferably higher than or equal to 10 percent per volume. This content is usually lower than or equal to 99.999 percent per volume, preferably lower than or equal to 99 percent per volume, more preferably lower than or equal to 95 percent per volume and most preferably preferably lower than or equal to 90 percent per volume. Without willing to be bound by any theory, it is believed that the combined presence of steam and hydrogen chloride may enhance the de-oligomerization and de-esterification reactions, and increase the recovery of valuable product in the stripping agent.

In the process according to the invention, the hydrogen chloride comprised in the stripping agent may be obtained from any process, like for instance in the manufacture of vinyl chloride or of 4,4-methylenediphenyl diisocyanate (MDI), or of toluene diisocyanate (TDI), or of hexamethylene diisocyanate (HMDI), or of allyl chloride by chlorinatioin of propylene, or of chloromethanes by chlorination of methane, or of chlorohydrofluorocarbons by hydrofluorination of chlorofluorocarbons or of hydrofluorocarbons by hydrofluorination of chloro- or chlorohydrofluorocarbons, or of chloroaromatics by chlorination of aromatics or of phosgene or of silica by flame hydrolysis of silicon tetrachloride or in the chlorinolysis of chlorinated organic compounds or in the high temperature oxidation of chlorinated compounds, or of any combination thereof.

In the process according to the invention, the hydrogen chloride comprised in the stripping agent may contain various types of impurities. These impurities may be organic compounds, inorganic compounds, or mixtures of them. The hydrogen chloride often contains organic compounds, and frequently aromatic organic compounds.

The impurity content of the hydrogen chloride is generally greater than or equal to 100 ppm by weight, usually greater than or equal to 1000 ppm by weight and often greater than or equal to 10 000 ppm by weight. This content is usually less than or equal to 10 % by weight, frequently less than or equal to 5 % by weight and in a lot of cases less than or equal to 2 % by weight.

The process according to the invention is advantageously carried out at any pressure compatible with keeping at least a major portion of the stripping agent in the gaseous state, often compatible with keeping the stripping agent t entirely in the gaseous state. This pressure is generally greater than or equal to 0.1 bar , often higher to 0.5 and more often greater than or equal to 1 bar. This pressure is usually less than or equal to 20 bar, frequently less than or equal to 15 bar, often less than or equal to 10 bar and in particular less than or equal to 5 bar. A pressure in the vicinity of 1 bar is also suitable.

The temperature at which the process is carried out is chosen so as to favor de-oligomerization reactions, and the subsequent conversion of heavy polymeric compounds into valuable easily strippable molecules. This temperature is preferably greater than or equal to 125°C, often greater than or equal to 130°C, frequently greater than or equal to 135°C and in a lot of cases greater than 140°C. This temperature is customarily less than or equal to 200°C, often less than or equal to 190°C and sometimes less than 180°C.

The ratio between the respective flows of liquid medium to be stripped and stripping agent is not critical and can vary to a large extent. It is in practice limited only by the cost of the possible regeneration of the stripping agent. The flow of stripping agent expressed as a weight percentage relative to the flow liquid medium to be stripped, is generally greater than or equal to 0.5, often greater than or equal to 1 and frequently greater than or equal to 2. This flow is usually less than or equal to 50, frequently less than or equal to 20 and often less than or equal to 10.

The ratio between the respective quantities of the liquid medium to be stripped and stripping agent is not critical and can vary to a large extent. It is in practice limited only by the cost of the possible regeneration of the stripping agent. The quantity of stripping agent, expressed as a weight percentage relative to the quantity of liquid medium to be stripped, is generally greater than or equal to 0.5, often greater than or equal to 1, frequently greater than or equal to 2, and in particular greater than or equal to 10. This quantity is usually less than or equal to 80, frequently less than or equal to 60, often less than or equal to 40 and in particular less than or equal to 20.

The process according to the invention may be carried out in continuous mode, semi-continuous mode or batch mode. The expression "continuous mode" is understood to denote an operating mode which is continuous for the stripping agent and continuous for the liquid medium. The expression "semi-continuous mode" is understood to denote an operating mode which is continuous for the stripping agent and in batch mode for the liquid liquid medium. The expression "batch mode" is understood to denote an operating mode which is in batch mode for the stripping agent and in batch mode for the liquid medium. It is preferred to operate in semi-continuous or continuous mode. Continuous mode is preferred.

When the process is carried out in batch mode, the duration of the contacting step between the liquid medium to be stripped and the stripping agent is generally greater than or equal to 0.01 min, often greater than or equal to 0.02 min and frequently greater than or equal to 0.05 min. This duration is usually less than or equal to 240 min, commonly less than or equal to 120 min, and frequently less than or equal to 90 min.

When the process is carried out in continuous mode, the residence time of the stripping agent during the contacting between the liquid medium to be stripped and the stripping agent is generally greater than or equal to 1 s, often greater than or equal to 2 s and frequently greater than or equal to 3 s. This residence time is usually less than or equal to 120 s, commonly less than or equal to 90 s, and frequently less than or equal to 60 s.

When the process is carried out in continuous mode, the residence time of the liquid medium during the contacting step between the liquid medium to be purified and the stripping agent is generally greater than or equal to 10 s, often greater than or equal to 15 s and frequently greater than or equal to 20 s. This residence time is usually less than or equal to 60 min, commonly less than or equal to 50 min, and frequently less than or equal to 45 min.

When the process is carried out in semi-continuous mode, the residence time of the stripping agent during the contacting step between the liquid medium to be purified and the stripping agent is generally greater than or equal to 1 s, often greater than or equal to 2 s and frequently greater than or equal to 3 s. This residence time is usually less than or equal to 120 s, commonly less than or equal to 90 s, and frequently less than or equal to 60 s.

The process can comprise providing a first stream of the liquid medium and a second stream of the stripping agent to a stripping zone, wherein the flow of the first stream expressed as a weight percentage relative to the flow of the second stream is greater than or equal to 0.5 and lower than or equal to 50 %.

In a first variant of the first embodiment, the process according to the invention comprises recovering a vapour fraction comprising the stripping agent and at least one of glycerol, monochloropropanediol and dichloropropanol. The glycerol, monochloropropanediol and dichloropropanol may have been present in the liquid medium before stripping, or may have been formed during the stripping, or both.

In a first aspect of this first variant, at least one first part of the vapour fraction is submitted to a reaction with at least one of glycerol, monochloropropanediol and an ester thereof, in the possible presence of a catalyst, in order to obtain dichloropropanol. The catalyst is as described above.

In a second aspect of the first variant, at least one second part of the vapor fraction is submitted to a separation operation in order to recover a first portion containing most of the glycerol, monochloropropanediol, and dichloropropanol contained in the second part of the vapor fraction prior to the separation operation and a second portion containing most of the stripping agent contained in the second part of the vapor fraction prior to the separation operation. The separation operation can be of any type, like for instance condensation, scrubbing, absorption, or adsorption. Condensation is preferred.

The first and the second aspects of the first variant can be combined.

In this second aspect, at least one part of the second portion can submitted to a reaction with at least one of glycerol, monochloropropanediol and an ester thereof, in the possible presence of a catalyst, in order to obtain dichloropropanol. The catalyst is as described above.

In a first feature of this second aspect, when the first portion contains dichloropropanol, at least one part of the first portion is submitted to a dehydrochlorination reaction in order to obtain epichlorohydrin. The dehydrochlorination is preferably carried out by reaction with a basic agent, such as lime or caustic soda.

In a second feature of this second aspect, when the first portion contains dichloropropanol, at least another part of the first portion is submitted to a reaction with a compound containing at least one active hydrogen atom in order to obtain an epoxy derivative selected from the group consisting of epoxy resins, glycidyl ethers, glycidyl esters, glycidyl amides, glycidyl imides, glycidyl amines, products that can be used as coagulants, wet-strength resins, cationization agents, flame retardants, ingredients for detergents, and any mixture of at least two of them. The reaction is preferably carried out in the presence of a basic agent, such as lime or caustic soda.

In a second embodiment, the invention is also related to a process for producing dichloropropanol by reacting at least one of glycerol, monochloropropanediol and an ester thereof, with a chlorinating agent comprising hydrogen chloride, in the possible presence of a catalyst, wherein a liquid medium containing at least one oligomer of glycerol possibly partially chlorinated and/or esterificated, is obtained, and wherein said liquid medium is submitted to a stripping process wherein a stripping agent comprising hydrogen chloride is used at a temperature higher than 120 °C.

In a third embodiment, the invention is also related to 1,3-dichloropropran-2-ol containing at least one other organic compound selected in the group consisting of acetaldehyde, vinyl chloride, ethyl chloride, acrolein, acetone, allyl chloride, allylic alcohol, acetic acid, 2-butanone, trichloromethane, 2-chloro-ethanol, hydroxyacetone, chloroacetone, propionic acid, 2,3-pentanedione, C3H7ClO, epichlorohydrin, (Z)-1,3-dichloropropene, 2-chloro-2-propen-1-ol, (E)-1,3-dichloropropene, 1,3-dichloropropane, cyclopentanone, 3-chloro-1-propanol, C4H7O2Cl (2 isomers), 2-methyl-2-cyclopentene-1-one, chlorobenzene, 1,2,3-trichloropropane, C₆H₁₂O, hexanoic acid, phenol, 2-acetoxy-1,3-dichloropropane, 1-acetoxy-2,3-dichloropropane, C₆H₈O₂Cl₂ (2 isomers), 1,3-dichloropropan-2-ol propionate, 2,3-dichloropropan-1-ol propionate, octanoic acid, C₆H₉Cl₃O₂, 1,3-dichloropropan-2-ol butyrate, C₆H₁₀O₂Cl₂ (at least 5 isomers), 2-hydroxy-3-methyl-acetophenone, C₆H₁₂Cl₂O₃, 1,3-dichloropropan-2-ol hexanoate, 1,3-dichloropropan-2-ol octanoate, 1-chloropropan-2-ol, 1-methoxy-3-chloropropan-2-ol, 2,3-dichloropropan-1-ol, 1-chloro-2,3-propanediol, 2-chloro-1,3-propanediol and glycerol. The preferred amounts of those organic compounds in the 1,3-dichloropropan-2-ol are given in Table 1. Said 1,3-dichloropropan-2-ol is obtainable by the process for making dichloropropanol according to the second embodiment of the invention.

**Table 1**

| Preferred Concentrations of organic compounds in thel,3-dichloropropran-2-ol in mg/kg | | | | | | |
|---|---|---|---|---|---|---|
| Organic compound | Preferred minima | | | Preferred maxima | | |
| Acetaldehyde | 0 | 0,1 | 0,5 | 30 | 60 | 300 |
| Vinyl Chloride | 0 | 1 | 7 | 25 | 40 | 200 |
| Ethyl Chloride | 0 | 0,1 | 1 | 10 | 20 | 100 |
| Acroleine | 0 | 1 | 60 | 300 | 400 | 2000 |
| Acetone | 0 | 10 | 100 | 800 | 1500 | 7500 |
| Allyl Chloride | 0 | 0,1 | 1 | 5 | 10 | 50 |
| Allylic Alcohol | 0 | 0,1 | 1 | 5 | 10 | 50 |
| Acetic Acid | 0 | 2 | 20 | 80 | 500 | 2500 |
| 2-butanone | 0 | 0,1 | 0,5 | 20 | 50 | 250 |
| Trichloromethane | 0 | 1 | 5 | 10 | 50 | 250 |
| 2-chloro-ethanol | 0 | 1 | 5 | 30 | 50 | 250 |
| Hydroxyacetone | 0 | 80 | 150 | 800 | 1500 | 7500 |
| Chloroacetone | 0 | 10 | 70 | 300 | 400 | 2000 |
| Propionic Acid | 0 | 1 | 5 | 10 | 20 | 100 |
| 2,3-pentanedione | 0 | 1 | 4 | 100 | 150 | 750 |
| C3H7ClO | 0 | 1 | 4 | 100 | 150 | 750 |
| Epichlorohydrin | 0 | 10 | 30 | 100 | 200 | 1000 |
| (Z)-1,3-dichloropropene | 0 | 1 | 2 | 30 | 50 | 250 |
| 2-chloro-2-propen-1-ol | 0 | 1 | 2 | 30 | 50 | 250 |
| (E)-1,3-dichloropropene | 0 | 1 | 2 | 10 | 50 | 250 |
| 1,3-dichloropropane | 0 | 1 | 2 | 700 | 1200 | 6000 |
| Cyclopentanone | 0 | 1 | 5 | 20 | 100 | 500 |
| 3-chloro-1-propanol | 0 | 10 | 100 | 1000 | 2000 | 10000 |
| C4H7O2Cl (2 isomers) | 0 | 1 | 2 | 5 | 10 | 50 |
| 2-methyl-2-cyclopentene-1-one | 0 | 1 | 2 | 20 | 100 | 500 |
| Chlorobenzene | 0 | 1 | 2 | 50 | 200 | 1000 |
| 1,2,3-trichloropropane | 0 | 2 | 20 | 500 | 800 | 4000 |
| C6H12O | 0 | 1 | 2 | 80 | 120 | 600 |
| Hexanoic Acid | 0 | 1 | 2 | 10 | 20 | 100 |
| Phenol | 0 | 1 | 6 | 90 | 120 | 600 |
| 2-acetoxy-1,3-dichloropropane | 0 | 1 | 10 | 30 | 50 | 250 |
| 1-acetoxy-2,3-dichloropropane | 0 | 1 | 2 | 30 | 50 | 250 |
| C6H8O2Cl2 (2 isomers) | 0 | 1 | 2 | 10 | 20 | 100 |
| 1,3-dichloropropan-2-ol propionate | 0 | 1 | 2 | 10 | 20 | 100 |
| 2,3-dichloropropan-1-ol propionate | 0 | 1 | 2 | 20 | 50 | 250 |
| Octanoic Acid | 0 | 1 | 2 | 20 | 50 | 250 |
| C6H9Cl302 | 0 | 1 | 2 | 10 | 30 | 150 |
| 1,3-dichloropropan-2-ol butyrate | 0 | 1 | 2 | 5 | 10 | 50 |
| C6H10O2Cl2 (at least 5 isomers) | 0 | 5 | 50 | 500 | 1000 | 5000 |
| 2-hydroxy-3-methyl-acetophenone | 0 | 1 | 2 | 1000 | 8000 | 40000 |
| C6H12Cl2O3 | 0 | 1 | 10 | 50 | 200 | 1000 |
| 1,3-dichloropropan-2-ol hexanoate | 0 | 1 | 2 | 10 | 40 | 200 |
| 1,3-dichloropropan-2-ol octanoate | 0 | 1 | 2 | 5 | 10 | 50 |
| 1-chloropropan-2-ol | 0 | 1 | 2 | 10 | 20 | 100 |
| 1-methoxy-3-chloropropan-2-ol | 0 | 1 | 2 | 10 | 40 | 200 |
| 1,3-dichloropropan-2-ol | 600000 | 700000 | 800000 | 980000 | 990000 | 999000 |
| 2,3-dichloropropan-1-ol | 1000 | 10000 | 30000 | 90000 | 12000 | 200000 |
| 1-chloro-2,3-propanediol | 0 | 1 | 10 | 200 | 10000 | 50000 |
| 2-chloro-1,3-propanediol | 0 | 1 | 10 | 100 | 3000 | 15000 |
| Glycerol | 0 | 1 | 2 | 100 | 1000 | 5000 |

In a fourth embodiment, the invention is furthermore related to a process for manufacturing epichlorohydrin, in which dichloropropanol is obtained by reacting at least one of glycerol, monochloropropanediol and an ester thereof, with a chlorinating agent comprising hydrogen chloride, in the possible presence of a catalyst, in which a liquid medium containing at least one of an oligomer of glycerol possibly partially chlorinated and/or esterificated is obtained, in which said liquid medium is submitted to a stripping process wherein a stripping agent comprising hydrogen chloride is used at a temperature higher than 120 °C, and in which said dichloropropanol is subjected to a dehydrochlorination reaction to produce epichlorohydrin.

In a fifth embodiment, the invention is also related to a process for manufacturing an epoxy derivative selected from the group consisting of epoxy resins, glycidyl ethers, glycidyl esters, glycidyl amides, glycidyl imides, glycidyl amines, products that can be used as coagulants, wet-strength resins, cationization agents, flame retardants, ingredients for detergents, epichlorohydrin elastomers, halogenated polyethers-polyols, monochloropropanediol, and any mixture of at least two of them, in which the epichlorohydrin produced by the process of the fourth embodiment is subjected to a reaction with at least one compound chosen from monoalcohols, monocarboxylic acids, polyols, polyamines, amino alcohols, polyimides, polyamides, polycarboxylic acids, ammonia, amines, polyaminoamides, polyimines, amine salts, phosphoric acid, phosphoric acid salts, phosphorus oxychlorides, phosphoric acid esters, phosphonic acids, esters of phosphonic acids, salts of phosphonic acids, phosphinic acids, esters of phosphinic acids, salts of phosphinic acids, phosphine oxides, phosphines, ethoxylated alcohols, alkylene or phenylene oxides, and mixtures of at least two of them, or in which the epichlorohydrin according to the invention is subjected to a homopolymerization reaction, or in which epichlorohydrin is subjected to a reaction of oligomerisation, of co-oligomerisation, of condensation, of dehydrochlorination and of hydrolysis, with water, or with a di- or polyhydroxylated compound which may optionally be halogenated and/or have ether oxide bonds and/or double bonds capable of being halogenated in a subsequent stage, or wherein epichlorohydrin is subjected to a reaction with water.

In a sixth embodiment, the invention is also finally related to a process for manufacturing an epoxy derivative selected from the group consisting of epoxy resins, glycidyl ethers, glycidyl esters, glycidyl amides, glycidyl imides, glycidyl amines, products that can be used as coagulants, wet-strength resins, cationization agents, flame retardants, ingredients for detergents, epichlorohydrin elastomers, halogenated polyethers-polyols, monochloropropanediol, and any mixture of at least two of them, in which the dichloropropanol produced by the process of the second embodiment,is subjected to a reaction with at least one compound chosen from monoalcohols, monocarboxylic acids, polyols, polyamines, amino alcohols, polyimides, polyamides, polycarboxylic acids, ammonia, amines, polyaminoamides, polyimines, amine salts, phosphoric acid, phosphoric acid salts, phosphorus oxychlorides, phosphoric acid esters, phosphonic acids, esters of phosphonic acids, salts of phosphonic acids, phosphinic acids, esters of phosphinic acids, salts of phosphinic acids, phosphine oxides, phosphines, ethoxylated alcohols, alkylene or phenylene oxides, and mixtures of at least two of them.

In a seventh embodiment, the invention is furthermore related to a process for manufacturing epichlorohydrin, in which 1,3-dichloropropan-2-ol according to the third embodiment is subjected to a dehydrochlorination reaction to produce epichlorohydrin.

In an eighth embodiment, the invention is also finally related to a process for manufacturing an epoxy derivative selected from the group consisting of epoxy resins, glycidyl ethers, glycidyl esters, glycidyl amides, glycidyl imides, glycidyl amines, products that can be used as coagulants, wet-strength resins, cationization agents, flame retardants, ingredients for detergents, epichlorohydrin elastomers, halogenated polyethers-polyols, monochloropropanediol, and any mixture of at least two of them, in which1,3-dichloropropan-2-ol according to the third embodiment, is subjected to a reaction with at least one compound chosen from monoalcohols, monocarboxylic acids, polyols, polyamines, amino alcohols, polyimides, polyamides, polycarboxylic acids, ammonia, amines, polyaminoamides, polyimines, amine salts, phosphoric acid, phosphoric acid salts, phosphorus oxychlorides, phosphoric acid esters, phosphonic acids, esters of phosphonic acids, salts of phosphonic acids, phosphinic acids, esters of phosphinic acids, salts of phosphinic acids, phosphine oxides, phosphines, ethoxylated alcohols, alkylene or phenylene oxides, and mixtures of at least two of them.

The examples below are intended to illustrate the invention without, however, limiting it.

### Example 1 (not according to the invention)

A liquid reaction medium in a glass thermostatised reactor has been stripped at about 142°C by a flow of gas introduced in the liquid via a fritted glass. The liquid has been stirred during the test with a magnetic barrel. The vaporized fraction has been weighted and analyzed after condensation at 0°C. The uncondensated fraction has been neutralized in a scrubber.

The liquid reaction medium at the beginning of the trial contained 141 g/kg of dichloropropanol, 90 g/kg of monochloropropanediol, 2 g/kg of glycerol, 0.5 g/kg of diglycerols, 4.9 g/kg of monochlorinated diglycerols, 54 g/kg of dichlorinated diglycerols, 2 g/kg of cyclic diglycerols, 9.5 g/kg of monochlorinated cyclic diglycerols, 8.1 g/kg of adipic acid, 5.6 g/kg of glycerol adipate, 64 g/kg of monochloropropanediol monoadipate, 17 g/kg of dichloropropanol monoadipate. The other compounds are essentially constituted of polyesters of adipic acid with glycerol, monochloropropanediol and/or dichloropropanol.

Nitrogen has been flushed at a constant flow rate of 1462 1N(normal liters) / h / kg of liquid medium. 111 g of dichloropropanol and 17 g of monochloropropanediol have been recovered in the condensate after the introduction of 1219 1N of gas for 1 kg of liquid reaction medium.

### Example 2 (according to the invention)

The trial has been realized with the same liquid reaction medium and in the same conditions as for example 1 except that nitrogen has been replaced by hydrogen chloride.

Hydrogen chloride has been flushed at a constant flow rate of 741 1N / h /kg of liquid medium. 143 g of dichloropropanol and 17 g of monochloropropanediol have been recovered in the condensate after the introduction of 1111 1N of gas for 1 kg of liquid reaction medium.

### Example 3 (not according to the invention)

The trial has been realized with the same liquid reaction medium and in the same conditions as for example 1 except that nitrogen has been replaced by a 1/1 vol/vol steam / nitrogen mixture.

The steam / nitrogen mixture has been flushed at a constant flow rate of 1370 1N / h / kg of liquid medium. 140 g of dichloropropanol and 24 g of monochloropropanediol have been recovered in the condensate after the introduction of 1141 1N of gas for 1 kg of liquid reaction medium.

### Example 4 (according to the invention)

The trial has been realized with the same liquid reaction medium and in the same conditions as for example 1 except that nitrogen has been replaced by a 1/1 vol/vol steam / hydrogen chloride mixture.

The steam / hydrogen chloride mixture has been flushed at a constant flow rate of 1365 1N / h / kg of liquid medium. 160 g of dichloropropanol and 16 g of monochloropropanediol have been recovered in the condensate after the introduction of 1137 1N of gas for 1 kg of liquid reaction medium.

## Claims

1. Process of stripping a liquid medium containing at least one oligomer of glycerol, wherein a stripping agent comprising hydrogen chloride is used at a temperature higher than 120 °C.

2. Process according to claim 1, wherein the oligomer of glycerol is chlorinated and/or esterificated.

3. Process according to claim 1 or 2, wherein the content of the at least one of the oligomer of glycerol, expressed as g of glycerol per kg of liquid medium is higher than or equal to 0.005 g/kg.

4. Process according to any one of claim 1 to 3, wherein the liquid medium comprises in addition at least one of glycerol, monochloropropanediol, dichloropropanol, and an ester thereof.

5. Process according to any one of claims 1 to 4, wherein the esters are esters of carboxylic acids.

6. Process according to any one of claims 1 to 5, wherein the stripping agent comprises in addition at least one compound selected form the group consisting of steam, methane, carbon dioxide, nitrogen, air, oxygen depleted air, and any mixture thereof.

7. Process according to any one of claims 1 to 6, exhibiting at least one of the following features:
● the stripping is carried out at a pressure between 0.1 and 20 bar absolute,
● the process comprises providing a first stream of the liquid medium and a second stream of the stripping agent to a stripping zone, wherein the flow of the first stream expressed as a weight percentage relative to the flow of the second stream is greater than or equal to 0.5 and lower than or equal to 50 %.

8. Process according to any one of claims 1 to 7, comprising recovering a vapor fraction comprising the stripping agent and at least one of glycerol, monochloropropanediol and dichloropropanol, and submitting at least one first part of the vapor fraction to a reaction with at least one of glycerol, monochloropropanediol, and an ester thereof, in the possible presence of a catalyst, in order to obtain dichloropropanol.

9. Process according to any of claims 1 to 7, comprising recovering a vapor fraction comprising the stripping agent and at least one of glycerol, monochloropropanediol and dichloropropanol, and submitting at least one second part of the vapor fraction to a separation operation in order to recover a first portion containing most of the glycerol, monochloropropanediol and dichloropropanol contained in the second part of the vapor fraction prior to the separation operation and a second portion containing most of the stripping agent contained in the second part of the vapor fraction prior to the separation operation.

10. Process according to claim 9, comprising submitting at least one part of the second portion to a reaction with at least one of glycerol, monochloropropanediol and an ester thereof, in the possible presence of a catalyst, in order to obtain dichloropropanol.

11. Process according to claim 9, wherein the first portion contains dichloropropanol and wherein at least one part of the first portion is further submitted to a dehydrochlorination reaction in order to obtain epichlorohydrin.

12. Process according to claim 9, wherein the first portion contains dichloropropanol and wherein at least one other part of the first portion is further submitted to a reaction with a compound containing at least one active hydrogen atom in order to obtain an epoxy derivative selected from the group consisting of epoxy resins, glycidyl ethers, glycidyl esters, glycidyl amides, glycidyl imides, glycidyl amines, products that can be used as coagulants, wet-strength resins, cationization agents, flame retardants, ingredients for detergents, and any mixture of at least two of them.

13. Process for producing dichloropropanol by reacting at least one of glycerol, monochloropropanediol and an ester thereof, with a chlorinating agent comprising hydrogen chloride, in the possible presence of a catalyst, wherein a liquid medium containing at least one of an oligomer of glycerol, possibly chlorinated and/or esterificated is obtained, and wherein said liquid medium is submitted to a stripping process according to any one of claims 1 to 12.

14. Process for manufacturing epichlorohydrin, in which the process according to Claim 12 is carried out in order to obtain dichloropropanol and in which said dichloropropanol is subjected to a dehydrochlorination reaction to produce epichlorohydrin.

15. Process for manufacturing an epoxy derivative selected from the group consisting of epoxy resins, glycidyl ethers, glycidyl esters, glycidyl amides, glycidyl imides, glycidyl amines, products that can be used as coagulants, wet-strength resins, cationization agents, flame retardants, ingredients for detergents, epichlorohydrin elastomers, halogenated polyethers-polyols, monochloropropanediol, and any mixture of at least two of them, comprising the process according to Claim 14, in which the epichlorohydrin is subjected to a reaction with at least one compound chosen from monoalcohols, monocarboxylic acids, polyols, polyamines, amino alcohols, polyimides, polyamides, polycarboxylic acids, ammonia, amines, polyaminoamides, polyimines, amine salts, phosphoric acid, phosphoric acid salts, phosphorus oxychlorides, phosphoric acid esters, phosphonic acids, esters of phosphonic acids, salts of phosphonic acids, phosphinic acids, esters of phosphinic acids, salts of phosphinic acids, phosphine oxides, phosphines, ethoxylated alcohols, alkylene or phenylene oxides, and mixtures of at least two of them, or in which the epichlorohydrin according to the invention is subjected to a homopolymerization reaction, or in which epichlorohydrin is subjected to a reaction of oligomerisation, of co-oligomerisation, of condensation, of dehydrochlorination and of hydrolysis, with water, or with a di- or polyhydroxylated compound which may optionally be halogenated and/or have ether oxide bonds and/or double bonds capable of being halogenated in a subsequent stage, or wherein epichlorohydrin is subjected to a reaction with water, or comprising the process according to Claim 13, in which the dichloropropanol is subjected to a reaction with at least one compound chosen from monoalcohols, monocarboxylic acids, polyols, polyamines, amino alcohols, polyimides, polyamides, polycarboxylic acids, ammonia, amines, polyaminoamides, polyimines, amine salts, phosphoric acid, phosphoric acid salts, phosphorus oxychlorides, phosphoric acid esters, phosphonic acids, esters of phosphonic acids, salts of phosphonic acids, phosphinic acids, esters of phosphinic acids, salts of phosphinic acids, phosphine oxides, phosphines, ethoxylated alcohols, alkylene or phenylene oxides, and mixtures of at least two of them.
